# EUROPEAN PATENT APPLICATION

(11) **EP 4 830 700 A1**
(43) Date of publication of application: **28.10.2026**
(21) Application number: 25172091.8
(22) Date of filing: 23.04.2025
(51) Int. Cl.: A61K 9/08, A61K 47/12, A61K 47/18

(54) **READY-TO-USE OXYTOCIN COMPOSITION**

(71) Applicant: B. Braun Melsungen AG, 34212 Melsungen (DE)
(72) Inventor: SCHWEBEL, Herve, 34212 Melsungen (DE); POITEVIN DUBOIS, Martine Bathilde, 34212 Melsungen (DE); ADAMO, Vincent, 34212 Melsungen (DE)
(74) Representative: Maiwald GmbH

(57) **Abstract**

The present disclosure is directed to a pharmaceutical composition comprising oxytocin, a stabilizer, and an aqueous medium, as well as to methods of manufacturing such pharmaceutical composition, and a unit dosage form comprising said pharmaceutical composition. Specifically, the pharmaceutical composition as disclosed herein is a ready-to-use oxytocin composition having an extended shelf life upon storage.

## Description

### Technical Field

The present disclosure is directed to a pharmaceutical composition comprising oxytocin, a stabilizer, and an aqueous medium, as well as to a method of manufacturing such pharmaceutical composition, and a unit dosage form comprising said pharmaceutical composition.

### Background of the invention

Oxytocin is a nonapeptide with two cysteine residues that form a disulfide bridge between positions one and six (SEQ ID NO.: 1). It is an extremely short-lived, fast acting hormone produced in the hypothalamus and stored in the posterior pituitary. From the posterior pituitary, oxytocin may be released into the bloodstream as a hormone in response to sexual activity or during childbirth.

Oxytocin is also available pharmaceutical forms. In either form (endogenous or exogenous), oxytocin stimulates uterine contractions to speed up the process of childbirth, i.e. it initiates or improves uterine contractions.

Oxytocin is currently indicated for stimulation of uterine contraction to induce labor, for the control of post-partum hemorrhage (PPH) following delivery of the placenta and for stimulation of lactation for breast-feeding. Currently, oxytocin is available in the United States as a single use 1 mL and multidose 10 mL and 50 mL glass vial. The formulation is typically present at a concentration of 10 international units (IU) per mL (IU/mL) and contains chlorobutanol at 0.5 % (w/v) (i.e. 5 mg/mL) as a preservative.

Typically, said formulation requires an up to 1000-fold dilution to a concentration of about 10 IU/L (or 10 milli international units per mL) prior to administration to a patient.

Thus, one of the drawbacks of the existing oxytocin formulations is that a dilution step is required prior to administration to a patient, thus increasing the complexity of treatment.

Furthermore, misdosing or dose wasting can occur while withdrawing medication from a vial as the formulation must be taken out of the vial with a syringe and has to be diluted to a concentration suitable for administration to the patient. This may lead to dose variation if there is any spillage from the syringe. Also while puncturing the vial, it is possible that small rubber pieces may enter the solution and may be administered unnoticed which can cause serious harm to patients.

Additionally, a dilution step delays the initiation of treatment, such as during emergency situations, and increases the risk of medication errors.

Further, after initial opening of a unit dosage form comprising said oxytocin formulation and dilution, the diluted composition decomposes rapidly and hence must be used within a short period of time.

Another drawback of the existing formulation is that it uses chlorobutanol as a preservative. However, chlorobutanol is a skin irritant and is also highly toxic to the liver.

A further problem of known formulations of oxytocin is their low storage stability when provided with concentrations of oxytocin that are suitable for administration to a patient without dilution.

### Summary of the invention

In view of the above, there is still a need for pharmaceutical compositions comprising oxytocin that overcome the above drawbacks. In particular, the present disclosure provides for a pharmaceutical composition of oxytocin that does not contain chlorobutanol as a preservative, that is ready-to-use without the need for dilution, and is storage stable, such as storage stable for at least 12 months.

Thus, according to a first aspect, the present disclosure relates to a pharmaceutical composition comprising a) oxytocin; b) a stabilizer, wherein the stabilizer is selected from the group consisting of i) lysine or a pharmaceutically acceptable salt thereof, ii) lactic acid or a pharmaceutically acceptable salt thereof, iii) citric acid or a pharmaceutically acceptable salt thereof, and iv) a mixture thereof; and c) an aqueous medium.

In a second aspect, the present disclosure relates to a method of manufacturing a pharmaceutical composition according to the first aspect of the present disclosure, the method comprising the steps of providing an aqueous medium, dissolving the stabilizer in said aqueous medium to provide a stabilizer solution, adjusting the pH of the stabilizer solution to a pH in the range of from about 3.4 to about 6.0 to provide an acidified stabilizer solution, dissolving oxytocin in said acidified stabilizer solution to provide an oxytocin solution, and sterilizing said oxytocin solution to provide a pharmaceutical composition according to the first aspect of the present disclosure.

According to a third aspect, the present disclosure relates to a pharmaceutical composition prepared by the method according to the second aspect of the present disclosure.

According to a fourth aspect, the present disclosure relates to a unit dosage form comprising a pharmaceutical composition according to the first aspect of the present disclosure or a pharmaceutical composition prepared by the method according to the third aspect of the present disclosure.

According to a fifth aspect, the present disclosure relates to a pharmaceutical composition according to the first aspect of the present disclosure or according to the third aspect of the present disclosure for use in medicine, such as for use in a method of initiation or improvement of uterine contractions.

### Detailed description of the invention

### The pharmaceutical composition

In a first aspect, the present disclosure relates to a pharmaceutical composition comprising a) oxytocin; b) a stabilizer, wherein the stabilizer is selected from the group consisting of i) lysine or a pharmaceutically acceptable salt thereof, ii) lactic acid or a pharmaceutically acceptable salt thereof, iii) citric acid or a pharmaceutically acceptable salt thereof, and iv) a mixture thereof; and c) an aqueous medium.

According to a preferred embodiment of the present disclosure, the pharmaceutical composition according to the first aspect of the present disclosure is a solution of oxytocin, the stabilizer, and optionally further components in the aqueous medium. According to another preferred embodiment of the first aspect of the present disclosure, the pharmaceutical composition is suitable for intravenous and/or intramuscular administration to a human patient. In that, the pharmaceutical composition according to the first aspect of the present disclosure is preferably in the form of a ready-to-use formulation. In other words, the pharmaceutical composition according to the first aspect of the present disclosure is suitable for administration to a human patient without prior dilution.

### Oxytocin

According to the first aspect of the present disclosure, the pharmaceutical composition comprises oxytocin. It is understood that oxytocin is the active pharmaceutical ingredient (API) of the pharmaceutical compositions as disclosed herein.

According to a preferred embodiment of the first aspect of the present disclosure, the pharmaceutical composition comprises oxytocin in a concentration of about 5 IU/L or more. According to another preferred embodiment of the first aspect of the present disclosure, the pharmaceutical composition comprises oxytocin in a concentration of about 10 IU/L or more. According to a further preferred embodiment of the first aspect of the present disclosure, the pharmaceutical composition comprises oxytocin in a concentration of about 12 IU/L or more.

The above concentrations of oxytocin may be suitable for direct administration to a patient in need thereof without dilution.

According to a preferred embodiment of the first aspect of the present disclosure, the pharmaceutical composition comprises oxytocin in a concentration of about 200 IU/L or less. According to another preferred embodiment of the first aspect of the present disclosure, the pharmaceutical composition comprises oxytocin in a concentration of about 100 IU/L or less. According to another preferred embodiment of the first aspect of the present disclosure, the pharmaceutical composition comprises oxytocin in a concentration of about 60 IU/L or less. According to a further preferred embodiment of the first aspect of the present disclosure, the pharmaceutical composition comprises oxytocin in a concentration of about 50 IU/L or less.

If the concentration of oxytocin in the pharmaceutical composition exceeds the above values, a dilution step may be needed before administration of the pharmaceutical composition according to the present disclosure to a patient in need thereof. Thus, the preparation of a formulation for injection is more complex and prone to medication errors as described hereinabove, and does not allow for use in emergency situations.

According to a preferred embodiment of the first aspect of the present disclosure, the pharmaceutical composition comprises oxytocin in a concentration in the range of from about 5 IU/L to about 200 IU/L. According to another preferred embodiment of the first aspect of the present disclosure, the pharmaceutical composition comprises oxytocin in a concentration in the range of from about 5 IU/L to about 100 IU/L. According to another preferred embodiment of the first aspect of the present disclosure, the pharmaceutical composition comprises oxytocin in a concentration in the range of from about 10 IU/L to about 60 IU/L. According to another preferred embodiment of the first aspect of the present disclosure, the pharmaceutical composition comprises oxytocin in a concentration in the range of from about 12 IU/L to about 60 IU/L. According to a further preferred embodiment of the first aspect of the present disclosure, the pharmaceutical composition comprises oxytocin most preferably in a concentration in the range of from about 12 IU/L to about 50 IU/L.

Pharmaceutical compositions comprising oxytocin in the above given concentration are particularly suitable for administration to a patient in need thereof without prior dilution. In other words, a pharmaceutical composition comprising oxytocin in the above given concentration is a ready-to-use oxytocin formulation and can be administered without preparation in emergency situations.

As is shown in examples 2 and 3 of the present disclosure, the pharmaceutical compositions as disclosed herein not only comprise oxytocin in a ready-to-use concentration but the inventors surprisingly found that oxytocin is highly stable upon storage of the pharmaceutical composition. In that, the inventors of the present disclosure surprisingly found that the ready-to-use pharmaceutical compositions of oxytocin according to the present disclosure have a shelf life upon storage of at least 12months.

### Stabilizer

According to the first aspect of the present disclosure, the pharmaceutical composition comprises a stabilizer.

The inventors of the present disclosure have surprisingly found that providing oxytocin in a liquid composition comprising a stabilizer as disclosed herein improves the storage stability and allows to successfully prepare ready-to-use formulations.

Accordingly, the stabilizer is selected from the group consisting of i) lysine or a pharmaceutically acceptable salt thereof, ii) lactic acid or a pharmaceutically acceptable salt thereof, iii) citric acid or a pharmaceutically acceptable salt thereof, and iv) any mixture thereof.

It is understood that the stabilizer(s) according to the present disclosure has a carboxylic acid group. Therefore, the presence of a pharmaceutically acceptable salt of lactic acid and/or lysine and/or citric acid or the acid form of lactic acid and/or lysine and/or citric acid, respectively, depends on the pH of the pharmaceutical composition. Hence, it is understood that depending on the pH of the pharmaceutical composition, the stabilizer may be in its acid form, its salt form, or in a mixture of its acid and its salt form. In other words, the stabilizer according to the present disclosure may act as a buffer system.

According to a preferred embodiment of the first aspect of the present disclosure, the stabilizer is lactic acid or a pharmaceutically acceptable salt thereof. According to another preferred embodiment of the first aspect of the present disclosure, the stabilizer is a mixture of lactic acid or a pharmaceutically acceptable salt thereof and citric acid or a pharmaceutically acceptable salt thereof.

According to another preferred embodiment of the first aspect of the present disclosure, the stabilizer is citric acid or a pharmaceutically acceptable salt thereof.

According to a further preferred embodiment of the first aspect of the present disclosure, the stabilizer is lysine or a pharmaceutically acceptable salt thereof. When the stabilizer is lysine, lysine may be present as one or both enantiomers of lysine, i.e. as L-lysine (also referred to as "(S)-lysine"), or as D-lysine (also referred to as "(R)-lysine"), or as a mixture of L-lysine and D-lysine.

According to a further preferred embodiment of the first aspect of the present disclosure, the stabilizer is a mixture of lysine or a pharmaceutically acceptable salt thereof and lactic acid or a pharmaceutically acceptable salt thereof. According to a further preferred embodiment of the first aspect of the present disclosure, the stabilizer is a mixture of lysine or a pharmaceutically acceptable salt thereof and citric acid or a pharmaceutically acceptable salt thereof. According to a further preferred embodiment of the first aspect of the present disclosure, the stabilizer is a mixture of lysine or a pharmaceutically acceptable salt thereof and lactic acid or a pharmaceutically acceptable salt thereof and citric acid or a pharmaceutically acceptable salt thereof.

The inventors of the present disclosure have surprisingly found that ready-to-use formulations of oxytocin having a superior storage stability are obtained when using the above preferred stabilizers. As evidenced in example 2, compositions comprising the above stabilizer only show minor degradation of oxytocin during storage at 40 °C, i.e. under accelerated storage conditions. In that, also the formation of impurities C and I is substantially reduced. In particular, when the stabilizer comprises lysine or a pharmaceutically acceptable salt thereof (i.e. lysine or a mixture comprising lysine), the storage stability is particularly high.

According to another preferred embodiment of the first aspect of the present disclosure, the pharmaceutical composition comprises the stabilizer in a concentration of about 0.5 mM or more. According to another preferred embodiment of the first aspect of the present disclosure, the pharmaceutical composition comprises the stabilizer in a concentration of about 1.0 mM or more. According to another preferred embodiment of the first aspect of the present disclosure, the pharmaceutical composition comprises the stabilizer in a concentration of about 2.0 mM or more. According to another preferred embodiment of the first aspect of the present disclosure, the pharmaceutical composition comprises the stabilizer in a concentration of about 3.0 mM or more. According to a further preferred embodiment of the first aspect of the present disclosure, the pharmaceutical composition comprises the stabilizer in a concentration of about 4.0 mM or more. According to a yet further preferred embodiment of the first aspect of the present disclosure, the pharmaceutical composition comprises the stabilizer in a concentration of about 4.5 mM or more.

The inventors of the present disclosure have found that the above minimum amount of stabilizer is required to stabilize oxytocin in solution.

According to a preferred embodiment of the first aspect of the present disclosure, the pharmaceutical composition comprises the stabilizer in a concentration of about 50 mM or less. According to another preferred embodiment of the first aspect of the present disclosure, the pharmaceutical composition comprises the stabilizer in the concentration of about 30 mM or less. According to another preferred embodiment of the first aspect of the present disclosure, the pharmaceutical composition comprises the stabilizer in the concentration of about 20 mM or less. According to another preferred embodiment of the first aspect of the present disclosure, the pharmaceutical composition comprises the stabilizer in the concentration of about 10 mM or less. According to a further preferred embodiment of the first aspect of the present disclosure, the pharmaceutical composition comprises the stabilizer in the concentration of about 8.0 mM or less. According to a yet further preferred embodiment of the first aspect of the present disclosure, the pharmaceutical composition comprises the stabilizer in the concentration of about 6.0 mM or less.

The inventors of the present disclosure have found that the above maximum concentration of stabilizer is sufficient to stabilize oxytocin solution. Furthermore, when exceeding the above maximum concentrations of stabilizer, the buffering effect of the stabilizer may be too high upon administration causing injection site irritations.

According to a preferred embodiment of the first aspect of the present disclosure, the pharmaceutical composition comprises the stabilizer in a concentration in the range of from about 0.5 mM to about 50 mM. According to another preferred embodiment of the first aspect of the present disclosure, the pharmaceutical composition comprises the stabilizer in a concentration in the range of from about 1.0 mM to about 30 mM. According to another preferred embodiment of the first aspect of the present disclosure, the pharmaceutical composition comprises the stabilizer in a concentration in the range of from about 2.0 mM to about 20 mM. According to another preferred embodiment of the first aspect of the present disclosure, the pharmaceutical composition comprises the stabilizer in a concentration in the range of from about 3.0 mM to about 10 mM. According to a further preferred embodiment of the first aspect of the present disclosure, the pharmaceutical composition comprises the stabilizer in a concentration in the range of from about 4.0 mM to about 8.0 mM. According to a yet further preferred embodiment of the first aspect of the present disclosure, the pharmaceutical composition comprises the stabilizer in a concentration in the range of from about 4.5 mM to about 6.0 mM. According to a most preferred embodiment of the first aspect of the present disclosure, the pharmaceutical composition comprises the stabilizer in a concentration of from about 5.0 mM.

As is shown in example 2 of the present disclosure, pharmaceutical compositions comprising oxytocin and a stabilizer as disclosed herein in the above given concentration are highly stable even opponent storage at 40 °C. Even starting the pharmaceutical composition comprising the stabilizer in the above given concentration for several months does not result in significant decomposition of oxytocin. Surprisingly, this effect is observed even in ready-to-use formulations.

In particular, it was found that the formation of impurity C and impurity I during storage is significantly reduced by providing oxytocin in a solution with a stabilizer as disclosed herein, preferably in an amount as disclosed herein.

It is understood that if the stabilizer is a mixture of two or three of lysine, lactic acid, and/or citric acids and/or respective pharmaceutically acceptable salts thereof, the concentration of the stabilizer as disclosed herein is the sum of the components. For example, if the pharmaceutical composition comprises lysine in a concentration of 2.0 mM, lactic acid in a concentration of 1.5 mM, and citric acid in a concentration of 0.25 mM, then the pharmaceutical composition according to the present disclosure comprises the stabilizer in a concentration of 3.75 mM.

### Aqueous medium

According to the first aspect of the present disclosure, the pharmaceutical composition on said comprises an aqueous medium, preferably an aqueous medium as defined herein.

The aqueous medium is preferably suitable for administration to a human. According to a preferred embodiment of the first aspect of the present disclosure, the aqueous medium is suitable for intravenous or intramuscular administration to a human patient.

Accordingly, it is understood that the aqueous medium is sterile, substantially free of pyrogens or free of pyrogens, and free of toxins.

It is understood that the aqueous medium serves as a solvent. In other words, the pharmaceutical composition as disclosed herein is preferably a solution of oxytocin, the stabilizer, and any further optional component as disclosed herein in the aqueous medium.

### Tonicity agent

According to a preferred embodiment of the first aspect of the present disclosure, the pharmaceutical composition further comprises a tonicity agent.

The tonicity agent may be advantageously added to adjust the tonicity of the pharmaceutical composition as disclosed herein. Therefore, the tonicity agent may be added in an amount or concentration that is adapted to make the pharmaceutical composition isotonic or bring the pharmaceutical composition close to isotonic tonicity. In other words, the pharmaceutical composition according to the present disclosure is an isotonic solution or a slightly hypertonic solution, or a slightly hypotonic solution, preferably an isotonic solution. Providing an isotonic solution of oxytocin is advantageous as it can be administered directly without dilution and with minimum or no injection site irritation.

According to a preferred embodiment of the first aspect of the present disclosure, the pharmaceutical composition has an osmolarity of about 600 mOsmol/L or less. According to another preferred embodiment of the first aspect of the present disclosure, the pharmaceutical composition has an osmolarity in the range of from about 32 mOsmol/L to about 600 mOsmol/L. According to another preferred embodiment of the first aspect of the present disclosure, the pharmaceutical composition has an osmolarity in the range of from about 100 mOsmol/L to about 600 mOsmol/L. According to another preferred embodiment of the first aspect of the present disclosure, the pharmaceutical composition has an osmolarity in the range of from about 120 mOsmol/L to about 450 mOsmol/L. According to another preferred embodiment of the first aspect of the present disclosure, the pharmaceutical composition has an osmolarity in the range of from about 150 mOsmol/L to about 450 mOsmol/L. According to another preferred embodiment of the first aspect of the present disclosure, the pharmaceutical composition has an osmolarity in the range of from about 200 mOsmol/L to about 400 mOsmol/L. According to a further preferred embodiment of the first aspect of the present disclosure, the pharmaceutical composition has an osmolarity in the range of from about 230 mOsmol/L to about 380 mOsmol/L. According to a yet further preferred embodiment of the first aspect of the present disclosure, the pharmaceutical composition has an osmolarity in the range of from about 250 mOsmol/L to about 350 mOsmol/L. According to a most preferred embodiment of the first aspect of the present disclosure, the pharmaceutical composition has an osmolarity in the range of from about 270 mOsmol/L to about 310 mOsmol/L.

According to a preferred embodiment of the first aspect of the present disclosure, the tonicity agent comprises a pharmaceutically acceptable alkali metal salt. Non-limiting examples of pharmaceutically acceptable alkali metal salts include an alkali metal chloride, alkali metal bromide, alkali metal acetate, alkali metal nitrate, alkali metal sulfate, alkali metal citrate, and the like. Non-limiting examples of alkali metals include lithium, sodium, and potassium. A particularly preferred alkali metal salt is sodium chloride.

According to another preferred embodiment of the first aspect of the present disclosure, the tonicity agent comprises a pharmaceutically acceptable earth alkali metal salt. Non-limiting examples of pharmaceutically acceptable earth alkali metal salts include an earth alkali metal chloride, earth alkali metal bromide, earth alkali metal acetate, earth alkali metal nitrate, earth alkali metal sulfate, earth alkali metal citrate, and the like. Non-limiting examples of earth alkali metals include magnesium, calcium, and beryllium. A particularly preferred earth alkali metal salt is calcium chloride.

According to another preferred embodiment of the first aspect of the present disclosure, the tonicity agent comprises a monosaccharide. Non-limiting examples of a monosaccharide include dextrose, fructose, galactose, ribose, deoxyribose, mannose, xylose, arabinose, allose, and talose. A particularly preferred monosaccharide is dextrose.

According to another preferred embodiment of the first aspect of the present disclosure, the tonicity agent comprises a mixture of a pharmaceutically acceptable alkali metal salt, and/or a pharmaceutically acceptable earth alkali metal salt, and/or a monosaccharide. According to a further preferred embodiment of the first aspect of the present disclosure, the tonicity agent comprises a mixture of at least one pharmaceutically acceptable alkali metal salt and at least one pharmaceutically acceptable earth alkali metal salt, preferably the tonicity agent is Ringer's lactate.

As used herein, "Ringer's lactate" is a mixture comprising sodium ions, potassium ions, calcium ions, chloride ions, and lactate ions. Accordingly, in a preferred embodiment of the first aspect of the present disclosure, the pharmaceutical composition comprises a tonicity agent comprising sodium ions (preferably in a concentration of about 130 mM), potassium ions (preferably in a concentration of about 4 mM), calcium ions (preferably in a concentration of about 2.7 mM), chloride ions (preferably in a concentration of about 109 mM), and lactate ions (preferably in a concentration of about 28 mM).

According to another preferred embodiment of the first aspect of the present disclosure, the tonicity agent is selected from the group consisting of sodium chloride, potassium chloride, sodium lactate, calcium chloride, dextrose, and mixtures thereof. According to a further preferred embodiment of the first aspect of the present disclosure, is sodium chloride, or Ringer's lactate, or dextrose. According to the most preferred embodiment of the first aspect of the present disclosure, the tonicity agent is sodium chloride.

As evidenced in examples 2 and 3 of the present disclosure, the inventors of the present disclosure have surprisingly found that the above tonicity agents further stabilize oxytocin in the pharmaceutical composition according to the present disclosure.

### pH of the pharmaceutical composition

According to a preferred embodiment of the first aspect of the present disclosure, the pharmaceutical composition has a pH that is suitable for administering the pharmaceutical composition to a human patient via the intravenous and/or intramuscular route. Hence, it is preferable that the pH of the pharmaceutical composition is in the range of from about 3.4 to about 6.0.

According to a preferred embodiment of the first aspect of the present disclosure, the pharmaceutical composition has a pH of about 3.4 or more. According to another preferred embodiment of the first aspect of the present disclosure, the pharmaceutical composition has a pH of about 3.6 or more. According to a further preferred embodiment of the first aspect of the present disclosure, the pharmaceutical composition has a pH of about 3.7 or more. According to a yet further preferred embodiment of the first aspect of the present disclosure, the pharmaceutical composition has a pH of about 3.8 or more. According to a most preferred embodiment of the first aspect of the present disclosure, the pharmaceutical composition has a pH of about 3.9 or more.

According to a preferred embodiment of the first aspect of the present disclosure, the pharmaceutical composition has a pH of about 6.0 or less. According to another preferred embodiment of the first aspect of the present disclosure, the pharmaceutical composition has a pH of about 5.0 or less. According to a further preferred embodiment of the first aspect of the present disclosure, the pharmaceutical composition has a pH of about 4.5 or less. According to a yet further preferred embodiment of the first aspect of the present disclosure, the pharmaceutical composition has a pH of about 4.3 or less. According to a most preferred embodiment of the first aspect of the present disclosure, the pharmaceutical composition has a pH of about 4.1 or less.

According to a preferred embodiment of the first aspect of the present disclosure, the pharmaceutical composition has a pH in the range of from about 3.4 to about 6.0. According to another preferred embodiment of the first aspect of the present disclosure, the pharmaceutical composition has a pH in the range of from about 3.6 to about 5.0. According to a further preferred embodiment of the first aspect of the present disclosure, the pharmaceutical composition has a pH in the range of from about 3.8 to about 4.5. According to a yet further preferred embodiment of the first aspect of the present disclosure, the pharmaceutical composition has a pH in the range of from about 3.9 to about 4.2. According to a most preferred embodiment of the first aspect of the present disclosure, the pharmaceutical composition has a pH of about 4.0.

The inventors of the present disclosure have surprisingly found that oxytocin is even more stable in solution with a stabilizer as disclosed herein when the pH is adjusted within the above given ranges. As is shown in examples 2 and 3 of the present disclosure, degradation of oxytocin upon storage at 40 °C is particularly slow. In that, the formation of impurity C and impurity I are considerably reduced.

Furthermore, the inventors surprisingly found that pharmaceutical compositions having a pH of around 4.0, such as having a pH in the range of from about 3.8 to about 4.5 or in the range of from about 3.9 to about 4.2, preferably a pH of about 4.0, are particularly stable upon storage, even under accelerated conditions. When the pH of the pharmaceutical composition according to the first aspect of the present disclosure is adjusted in said range (i.e., a pharmaceutical composition according to the first aspect of the present disclosure, wherein the pH is in the range of from about 3.8 to about 4.5 or in the range of from about 3.9 to about 4.2, preferably the pH is about 4.0), oxytocin is even more stable and decomposes only very slowly (see example 3). In that, also the formation of impurity C and impurity I are considerably reduced.

### Excluded components

According to a preferred embodiment of the first aspect of the present disclosure, the pharmaceutical composition does not comprise gluconic acid or a pharmaceutically acceptable salt thereof. According to another preferred embodiment of the first aspect of the present disclosure, the pharmaceutical composition does not comprise glutamic acid or a pharmaceutically acceptable salt thereof. According to another preferred embodiment of the first aspect of the present disclosure, the pharmaceutical composition does not comprise lactobionic acid or a pharmaceutically acceptable salt thereof. According to another preferred embodiment of the first aspect of the present disclosure, the pharmaceutical composition does not comprise sucrose.

The inventors of the present disclosure have surprisingly found that the stability of oxytocin during storage is significantly improved when using the stabilizer(s) as disclosed herein as opposed to gluconic acid, glutamic acid, and/or lactobionic acid, and/or sucrose.

Hence, according to another preferred embodiment of the first aspect of the present disclosure, the pharmaceutical composition does not comprise chlorobutanol, and/or gluconic acid or a pharmaceutically acceptable salt thereof, and/or glutamic acid or a pharmaceutically acceptable salt thereof, and/or lactobionic acid or a pharmaceutically acceptable salt thereof, and/or a sucrose.

According to another preferred embodiment of the first aspect of the present disclosure, the pharmaceutical composition does not comprise chlorobutanol. According to a further preferred embodiment of the present disclosure, the pharmaceutical composition does not comprise a preservative. The inventors of the present invention have surprisingly found that the pharmaceutical composition according to the present disclosure is storage stable in the absence of chlorobutanol or other preservative.

According to a further preferred embodiment of the first aspect of the present disclosure, the pharmaceutical composition does not comprise a C₄-C₁₂ carboxylic acid or pharmaceutically acceptable salt thereof. According to a further preferred embodiment of the first aspect of the present disclosure, the pharmaceutical composition does not comprise a disaccharide.

According to a further preferred embodiment of the present disclosure, the pharmaceutical composition does not comprise a C₄-C₁₂ carboxylic acid or pharmaceutically acceptable salt thereof, and/ or a disaccharide, and/or a sugar alcohol, and/or chlorobutanol

### Storage stability

According to another preferred embodiment of the present disclosure, the concentration of oxytocin in the pharmaceutical composition decreases by about 4 % per month during storage at 40 °C / 75 % rH, preferably determined according to formula (1).

In other words, the average loss of oxytocin per month (hereinafter referred to as "ALO") as defined herein may be about 4 % per month or less, preferably determined according to formula (1).

According to another preferred embodiment of the present disclosure, the pharmaceutical composition has an ALO of about 3.5 % per month or less, preferably determined according to formula (1). According to a further preferred embodiment of the present disclosure, the pharmaceutical composition has an ALO of about 3.0 % per month or less, preferably determined according to formula (1). According to a further preferred embodiment of the present disclosure, the pharmaceutical composition has an ALO of about 2.5 % per month or less, preferably determined according to formula (1).

According to another preferred embodiment of the present disclosure, the pharmaceutical composition has an average increase of impurity C (AIC) of about 3.0 % per month or less, preferably determined according to formula (2). According to a further preferred embodiment of the present disclosure, the pharmaceutical composition has an AIC of about 2.0 % per month or less, preferably determined according to formula (2). According to a further preferred embodiment of the present disclosure, the pharmaceutical composition has an AIC of about 1.0 % per month or less, preferably determined according to formula (2).

According to another preferred embodiment of the present disclosure, the pharmaceutical composition has an average increase of impurity I (All) of about 3.0 % per month or less, preferably determined according to formula (3). According to a further preferred embodiment of the present disclosure, the pharmaceutical composition has an All of about 2.0 % per month or less, preferably determined according to formula (3). According to a further preferred embodiment of the present disclosure, the pharmaceutical composition has an All of about 1.0 % per month or less, preferably determined according to formula (3).

As is evidenced in example 2 and 3 of the present disclosure, the pharmaceutical compositions according to the present disclosure are highly stable upon storage, even under accelerated conditions of 40 °C. It is understood that storage of a pharmaceutical composition at 40 °C for 1 month is equivalent to storage at 25 °C for 3 months (Arrhenius equation).

Also, the formation of at least impurities C and I is particularly slow.

### The method of manufacturing a pharmaceutical composition

In a second aspect, the present disclosure relates to a method of manufacturing a pharmaceutical composition according to the first aspect of the present disclosure, the method comprising the steps of providing an aqueous medium, dissolving the stabilizer in said aqueous medium to provide a stabilizer solution, adjusting the pH of the stabilizer solution to a pH in the range of from about 3.4 to about 6.0 to provide an acidified stabilizer solution, dissolving oxytocin in said acidified stabilizer solution to provide an oxytocin solution, and sterilizing said oxytocin solution to provide a pharmaceutical composition according to the first aspect of the present disclosure.

### Providing an aqueous medium

In a first step of the method according to the second aspect of the present disclosure, aqueous medium as disclosed herein is provided (also referred to as "step a)").

It is understood that said aqueous medium may be provided in any container that is suitable for performing the method according to the second aspect of the present disclosure.

According to a preferred embodiment of the second aspect of the present disclosure, the aqueous medium is provided in an amount of at least about 70 % (v/v) based on the volume of oxytocin solution provided in step d). According to another preferred embodiment of the second aspect of the present disclosure, the aqueous medium is provided in an amount of at least about 80 % (v/v) based on the volume of oxytocin solution provided in step d). According to a further preferred embodiment of the second aspect of the present disclosure, the aqueous medium is provided in an amount of at least about 90 % (v/v) based on the volume of oxytocin solution provided in step d).

According to a preferred embodiment of the second aspect of the present disclosure, the aqueous medium is provided in an amount of about 98 % (v/v) or less based on the volume of oxytocin solution provided in step d). According to another preferred embodiment of the second aspect of the present disclosure, the aqueous medium is provided in an amount of about 95 % (v/v) or based on the volume of oxytocin solution provided in step d). According to a further preferred embodiment of the second aspect of the present disclosure, the aqueous medium is provided in an amount of about 93 % (v/v) or less based on the volume of oxytocin solution provided in step d).

It is noted that the above given amounts of aqueous medium may be advantageous in the method according to the second aspect of the present disclosure as they allow to adjust the pH and concentrations of all components in a precise manner.

According to a preferred embodiment of the second aspect of the present disclosure, step a) is performed at about room temperature.

### Dissolving the stabilizer

In a second step, the stabilizer as defined herein is dissolved in said aqueous medium provided in step a) to provide a stabilizer solution. This step may also be referred to as "step b)".

Optionally, also the tonicity agent (if present) is dissolved in the aqueous medium provided in step a).

It is understood that the stabilizer solution thus provided comprises the stabilizer, the aqueous medium, and the tonicity agent (if present).

In that, the stabilizer solution comprises the stabilizer in a concentration that is higher than the concentration of the stabilizer in the pharmaceutical composition according to the first aspect of the present disclosure. However, the concentration of the stabilizer in the stabilizer solution is adapted in that the concentration of the stabilizer is diluted into the range as disclosed herein with regard to the first aspect of the present disclosure upon adding any remaining amount of aqueous medium in step d).

In other words, the concentration of the stabilizer in the stabilizer solution is about x-times the concentration of the stabilizer in the pharmaceutical composition according to the first aspect of the present disclosure, wherein x is the ratio of $x = \frac{\left.volume of oxytocin composition obtained in step d\right)}{volume of stabilizer composition} .$

According to a preferred embodiment of the second aspect of the present disclosure, step b) is performed at about room temperature.

According to a preferred embodiment of the second aspect of the present disclosure, step b) comprises dissolving a stabilizer as disclosed herein and a tonicity agent as disclosed herein in the aqueous medium a provided in step a) to provide a stabilizer solution.

In that, the stabilizer solution comprises the stabilizer and the tonicity agent in a concentration that is higher than the respective concentrations of the stabilizer and the tonicity agent in the pharmaceutical composition according to the first aspect of the present disclosure. However, the respective concentrations of the stabilizer and the tonicity agent in the stabilizer solution is adapted in that the concentration of the stabilizer is diluted into the range as disclosed herein with regard to the first aspect of the present disclosure upon adding any remaining amount of aqueous medium in step d).

In other words, the concentration of the stabilizer in the stabilizer solution and the concentration of the tonicity agent in the stabilizer solution are about x-times the concentration of the stabilizer in the pharmaceutical composition according to the first aspect of the present disclosure and the concentration of the tonicity agent in the pharmaceutical composition according to the first aspect of the present disclosure, respectively, wherein x is the ratio of $x = \frac{\left.volume of oxytocin composition obtained in step d\right)}{volume of stabilizer composition} .$

According to a preferred embodiment of the second aspect of the present disclosure, step b) is performed at about room temperature.

### Adjusting the pH

In a subject step of the method according to the second aspect of the present disclosure, the pH of the stabilizer solution provided in step b) is adjusted to a pH in the range of from about 3.4 to about 6.0 to provide an acidified stabilizer solution. This step is also referred to as "step c)".

According to a preferred embodiment of the second aspect of the present disclosure, the pH of the stabilizer solution is adjusted by adding a suitable amount of an inorganic acid and/or an inorganic base to the stabilizer solution, preferably by adding a solution of an inorganic acid in an aqueous medium as disclosed herein to the stabilizer solution and/or by adding a solution of an inorganic base in an aqueous medium as disclosed herein to the stabilizer solution. The concentration of the inorganic acid in the aqueous medium may preferably be about 0.1 M. The concentration of the inorganic base in the aqueous medium may preferably be about 0.1 M.

Non-limiting examples of such inorganic acid include HCI, sulfuric acid, and phosphoric acid. Non-limiting examples of inorganic basis include sodium hydroxide, potassium hydroxide, and calcium hydroxide.

According to a preferred embodiment of the second aspect of the present disclosure, the pH of the acidified stabilizer solution is the same pH as disclosed herein for the pharmaceutical composition according to the first aspect of the present disclosure. In other words:
According to a preferred embodiment of the second aspect of the present disclosure, the acidified stabilizer solution has a pH of about 3.4 or more. According to another preferred embodiment of the second aspect of the present disclosure, the acidified stabilizer solution has a pH of about 3.6 or more. According to a further preferred embodiment of the second aspect of the present disclosure, the acidified stabilizer solution has a pH of about 3.7 or more. According to a yet further preferred embodiment of the second aspect of the present disclosure, the acidified stabilizer solution has a pH of about 3.8 or more. According to a most preferred embodiment of the second aspect of the present disclosure, the acidified stabilizer solution has a pH of about 3.9 or more.

According to a preferred embodiment of the second aspect of the present disclosure, the acidified stabilizer solution has a pH of about 6.0 or less. According to another preferred embodiment of the second aspect of the present disclosure, the acidified stabilizer solution has a pH of about 5.0 or less. According to a further preferred embodiment of the second aspect of the present disclosure, the acidified stabilizer solution has a pH of about 4.5 or less. According to a yet further preferred embodiment of the second aspect of the present disclosure, the acidified stabilizer solution has a pH of about 4.3 or less. According to a most preferred embodiment of the second aspect of the present disclosure, the acidified stabilizer solution has a pH of about 4.1 or less.

According to a preferred embodiment of the second aspect of the present disclosure, the acidified stabilizer solution has a pH in the range of from about 3.4 to about 6.0. According to another preferred embodiment of the second aspect of the present disclosure, the acidified stabilizer solution has a pH in the range of from about 3.6 to about 5.0. According to a further preferred embodiment of the second aspect of the present disclosure, the acidified stabilizer solution has a pH in the range of from about 3.8 to about 4.5. According to a yet further preferred embodiment of the second aspect of the present disclosure, the acidified stabilizer solution has a pH in the range of from about 3.9 to about 4.2. According to a most preferred embodiment of the second aspect of the present disclosure, the acidified stabilizer solution has a pH of about 4.0.

The inventors of the present invention have surprisingly found, that adjusting the pH in the above range during manufacturing of an oxytocin solution increases the stability of oxytocin in the process of manufacturing.

According to another preferred embodiment of the second aspect of the present disclosure, step c) is performed at about room temperature.

### Dissolving oxytocin

In a fourth step of the method according to the second aspect of the present disclosure, oxytocin is dissolved in the acidified stabilizer solution provided in step c) to provide an oxytocin solution. This step is also referred to as "step d)".

According to another preferred embodiment of the second aspect of the present disclosure, step d) further comprises adding the remaining amount of aqueous medium. In other words, it is preferred to add a remaining amount of aqueous medium to the acidified stabilizer solution to the volume of oxytocin composition obtained in step d). In that, the remaining amount of aqueous medium added in step d) is adapted to dilute the acidified stabilizer solution provided in step c) so that the concentrations of oxytocin, stabilizer, and tonicity agent (if present) are the same as disclosed herein with respect to the pharmaceutical composition according to the first aspect of the present disclosure.

In other words, the concentration of oxytocin in the oxytocin solution provided in step d) of the method according to the second aspect of the present disclosure is the same as the concentration of oxytocin in the pharmaceutical composition according to the first aspect of the present disclosure.

The concentration of stabilizer in the oxytocin solution provided in step d) of the method according to the second aspect of the present disclosure is the same as the concentration of stabilizer in the pharmaceutical composition according to the first aspect of the present disclosure.

The concentration of tonicity agent (if present) are in the oxytocin solution provided in step d) of the method according to the second aspect of the present disclosure is the same as the concentration of tonicity agent (if present) in the pharmaceutical composition according to the first aspect of the present disclosure.

Thus, it is understood that the final volume of the pharmaceutical composition obtained by the method according to the second aspect of the present disclosure is determined in step d).

According to another preferred embodiment of the second aspect of the present disclosure, step d) further includes stirring the oxytocin solution, such as for a duration of at least 5 minutes, preferably for a duration of about 10 minutes.

According to another preferred embodiment of the second aspect of the present disclosure, step d) further comprises measuring the pH of the oxytocin solution and adjusting the pH of the oxytocin solution to the pH of the pharmaceutical composition as disclosed herein, if necessary. In other words, step d) may include checking and readjusting the pH of the oxytocin solution in the range as disclosed herein with regard to the pharmaceutical composition according to the first aspect of the present disclosure, if necessary.

### Sterilizing

In a fifths step of the method according to the second aspect of the present disclosure, the oxytocin solution provided in step d) is sterilized to provide a pharmaceutical composition according to the first aspect of the present disclosure. This step may also be referred to as "step e)".

Methods of sterilizing an oxytocin solution are known to the skilled person in the art and are not particularly limited. Nevertheless, it is understood that the step e) results in a pharmaceutical composition according to the first aspect of the present disclosure. In other words, the step of sterilizing the oxytocin solution provided in step d) may not change the composition of the oxytocin solution and yields a pharmaceutical composition that is suitable for administration to a human patient, preferably suitable for intravenous administration and/or intramuscular administration.

According to a preferred embodiment of the present disclosure, step e) comprises sterile filtration. Sterile filtration is known in the art and does not involve heating. Therefore, the oxytocin remains stable and no decomposition occurs.

According to another preferred embodiment of the second aspect of the present disclosure, step e) is performed at about room temperature.

### Filling

According to a preferred embodiment of the second aspect of the present disclosure, the method further comprises a step of filling the pharmaceutical composition according to the first aspect of the present disclosure into a container under aseptic conditions.

According to a further preferred embodiment of the second aspect of the present disclosure, the container is a syringe. According to a yet further preferred embodiment of the second aspect of the present disclosure, the container is an IV bag, such as a plastic bag.

According to another preferred embodiment of the second aspect of the present disclosure, the container may be placed in a secondary packaging. According to a further preferred embodiment of the second aspect of the present disclosure, the secondary packaging is a pouch, preferably a plastic patch. According to another preferred embodiment of the second aspect of the present disclosure, the secondary packaging is a bag, such as a plastic bag.

The secondary packaging does preferably not include an oxygen scavenger. Still, the pharmaceutical composition is highly stable as evidenced in example 2.

### The pharmaceutical composition prepared by a method disclosed herein

According to a third aspect, the present disclosure relates to a pharmaceutical composition prepared by the method according to the second aspect of the present disclosure.

It is understood that the pharmaceutical composition prepared by the method according to the second aspect of the present disclosure is characterized by the same features as the pharmaceutical composition according to the first aspect of the present disclosure.

### Unit dosage form

According to a fourth aspect, the present disclosure relates to a unit dosage form comprising a pharmaceutical composition according to the first aspect of the present disclosure or a pharmaceutical composition prepared by the method according to the third aspect of the present disclosure.

According to a preferred embodiment of the fourth aspect of the present disclosure, the unit dosage form is a container comprising the pharmaceutical composition according to the first aspect of the present disclosure or the pharmaceutical composition according to the third aspect of the present disclosure. In other words, in a fourth aspect, the present disclosure relates to a container comprising the pharmaceutical composition according to the first aspect of the present disclosure or the pharmaceutical composition according to the third aspect of the present disclosure.

According to a further preferred embodiment of the fourth aspect of the present disclosure, the container is a syringe. According to a yet further preferred embodiment of the fourth aspect of the present disclosure, the container is an IV bag.

The unit dosage form may be overwrapped with a secondary packaging, such as a box or a foil. The secondary packaging does preferably not include an oxygen scavenger. Still, the pharmaceutical composition is highly stable as evidenced in example 2.

### Therapeutic use

According to a fifth aspect, the present disclosure relates to a pharmaceutical composition according to the first aspect of the present disclosure or according to the third aspect of the present disclosure for use in medicine, such as for use in a method of initiation or improvement of uterine contractions.

In that, it is understood that the pharmaceutical composition according to the first aspect of the present disclosure or according to the third aspect of the present disclosure is directly used without prior dilution. Therefore, the pharmaceutical compositions as disclosed herein may be advantageously used in emergency situations.

### Definitions

As used herein, "oxytocin" refers to a peptide of SEQ ID No. 1. In other words, oxytocin is a peptide with the sequence **Cys-Tyr-Ile-Gln-Asn-Cys-Pro-Leu-Gly-NH₂,** wherein the two cysteine residues at positions 1 and 6 are connected by a disulfide bond. Oxytocin is also known under CAS number 50-56-6. Oxytocin has a molecular weight of 1007.19 Da.

Hence, it is understood that oxytocin as used herein is represented by the following formula:

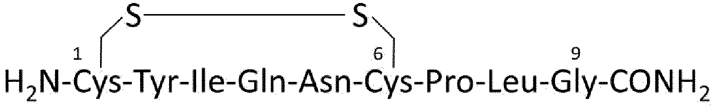

In yet other words, oxytocin has the following structure: 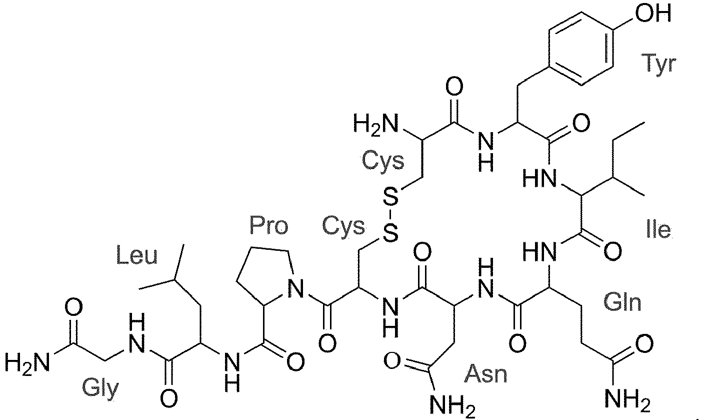 wherein all amino acids of oxytocin except for glycine are the respective L-amino acids.

As used herein, the term "IU" stands for "international unit of oxytocin" and 1 IU is the equivalent of 1.68 µg of oxytocin. As used herein, the concentration of oxytocin, e.g. in IU/L, is determined according to method (1) as disclosed herein.

As used herein, the term "impurity C" refers to a peptide of SEQ ID No. 2. In other words, impurity C is a derivative of oxytocin, wherein the glutamine at position 4 is deaminated to form glutamic acid. In yet other words, impurity C refers to a peptide with the sequence **Cys-Tyr-Ile-Glu-Asn-Cys-Pro-Leu-Gly-NH₂,** wherein the two cysteine residues at positions 1 and 6 are connected by a disulfide bond. Impurity C is also known under CAS number 4314-67-4. As used herein, the concentration of impurity C, e.g. in wt.-%, is determined according to method (1) as disclosed herein.

As used herein, the term "impurity I" refers to a peptide of SEQ ID No. 3. In other words, impurity I is a derivative of oxytocin, wherein the asparagine at position 5 is deaminated to form aspartic acid. In yet other words, impurity I refers to a peptide with the sequence **Cys-Tyr-Ile-Gln-Asp-Cys-Pro-Leu-Gly-NH₂,** wherein the two cysteine residues at positions 1 and 6 are connected by a disulfide bond. As used herein, the concentration of impurity I, e.g. in wt.-%, is determined according to method (1) as disclosed herein.

As used herein, "lysine" relates to a compound of the following formula:

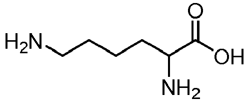

It is noted that lysine has a stereo center. In other words, "lysine" as used herein may comprise one or both enantiomers of lysine, i.e. L-lysine (also referred to as "*(S)*-lysine") and/or D-lysine (also referred to as "*(R)*-lysine").

As used herein, "L-lysine"(also known under CAS number 56-87-1) refers to a compound of the following structure:

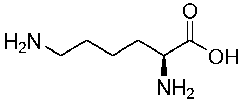

As used herein, "D-lysine" (also known under CAS number 923-27-3) refers to a compound of the following structure:

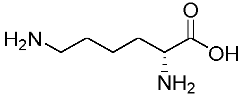

According to a preferred embodiment of the present disclosure, lysine comprises L-lysine and/or D-lysine. According to a further preferred embodiment of the present disclosure, lysine is L-lysine.

As used herein the terms "chlorobutanol" and "chlorbutanol" are used interchangeably and referred to trichloro-2-methyl-2-propanol. Chlorobutanol is also known under CAS number 57-15-8.

As used herein, the osmolarity of a pharmaceutical composition is determined according to European Pharmacopeia (Monograph Identifying Code 07/2019:20235 of Pharmacopoeia Europea 10.7).

As used herein, the term "comprise", and variations such as "comprises" and "comprising", will be understood to imply the inclusion of a stated member, integer or step but not the exclusion of any other non-stated member, integer or step, unless the context requires otherwise. The term "consist of" is a particular or preferred embodiment of the term "comprise", wherein any other non-stated member, integer or step is excluded. In the context of the present invention, the term "comprise" encompasses the term "consist of". The term "comprising" thus encompasses "including" as well as "consisting of" e.g, a composition "comprising" X may consist exclusively of X or may include something additional e.g., X + Y.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The word "substantially" does not exclude "completely" e.g., a composition which is "substantially free" from Y may be completely free from Y. Where necessary, the word "substantially" may be omitted from the definition of the invention.

The term "about" in relation to a numerical value x means x ± 20 %, preferably x ± 10 %, more preferably x ± 5 %, even more preferably x ± 2 %, and still more preferably x ± 1 %.

As used herein, the term "pharmaceutically acceptable salt" refers to an ionic assembly of cations and anions, which is composed of related numbers of cations and anions, so that the product (the pharmaceutically acceptable salt) is electrically neutral (without net charge). In that, it is understood that the skilled person may balance the charge of a component, such as an ascorbate ion, with a respective pharmaceutically acceptable cation or a pharmaceutically acceptable anion as needed.

As used herein, the term "pharmaceutically acceptable cation" refers to a cation that is not toxic to mammals. Preferred pharmaceutically acceptable cations are lithium, sodium, potassium, aluminium, and zinc, cations made from organic bases such as choline, diethanolamine, morpholine, ammonium salts, quaternary salts such as tetramethylammonium salt; amino acid addition salts such as salts with glycine and arginine.

As used herein, the term "pharmaceutically acceptable anion" refers to an anion that is not toxic to mammals. Said pharmaceutically acceptable anion may have one, two, three, or more negative charges. Preferred pharmaceutically acceptable anions are F⁻, Cl⁻, Br⁻, I⁻, OH⁻, tosylate, mesylate, triflate, trifluoroacetate, sulfate, acetate, tartrate, benzoate, oxylate, salicylate, or camphorsulfonate. Further preferred pharmaceutically acceptable anions are F⁻, Cl⁻, Br⁻, I⁻, or OH⁻. A particularly preferred pharmaceutically acceptable anion is Cl⁻.

As used herein, the term "room temperature" refers to a temperature of 25 °C. All reactions were performed at room temperature, unless explicitly stated otherwise.

As used herein, the term "aqueous medium" refers to a liquid medium comprising water as the primary component. The aqueous medium may include additional water-miscible cosolvents. Preferably, an aqueous medium as used herein comprises at least 70 % (v/v), preferably at least 80 % (v/v), more preferably at least 90 %(v/v), yet further preferably at least 95 % (v/v), even more preferably at least 98 % (v/v) of water, each based on the total volume of the aqueous medium. According to a further preferred embodiment, the aqueous medium consists of water. According to a most preferred embodiment, the aqueous medium consists of water for injection (WFI).

As used herein, "water for injection" which may be abbreviated "WFI" refers to a pharmaceutically acceptable, sterile, non-pyrogenic, and highly purified form of water that complies with pharmacopeial standards, preferably with the United States Pharmacopeia (USP) as in force on 1 April 2025 and/or the European Pharmacopeia (Ph. Eur.) as in force on 1 April 2025. As such, WFI is intended for use as a solvent in the preparation of parenteral solutions and other sterile products. WFI may be produced by distillation or reverse osmosis followed by ultrafiltration and may meet stringent limits for microbial contamination and total organic carbon (TOC), containing no added antimicrobial agents or other substances. WFI may be characterized by a conductivity not exceeding pharmacopeial limits, preferably according to the United States Pharmacopeia (USP) as in force on 1 April 2025 and/or the European Pharmacopeia (Ph. Eur.) as in force on 1 April 2025. WFI is typically used immediately after production or stored under validated conditions to maintain its quality.

As used herein, the term "disaccharide" refers to an organic compound consisting of two monosaccharides which are joined by glycosidic linkage. Said glycosidic linkage may be α-glycosidic, such as α(1→2), α(1→3), α(1→4), α(1→5), or α(1→6); or β-glycosidic, such as β(1→2), β(1→3), β(1→4), or β(1→6). Non-limiting examples of disaccharides are sucrose, lactose, maltose, trehalose, cellobiose, chitobiose, kojibiose, nigerose, isomaltose, sophorose, laminaribiose, gentiobiose, trehalulose, turanose, maltulose, leucrose, isomaltulose, gentiobiulose, mannobiose, melibiose, rutinose, rutinulose, and xylobiose.

As used herein, the "average loss of oxytocin per month" (that may be abbreviated as "ALO") is determined according to the formula (1) $ALO = \frac{{c}_{0} \left(oxytocin\right) - {c}_{t} \left(oxytocin\right)}{t}$ wherein "c₀(oxytocin)" is the concentration of oxytocin in a pharmaceutical composition in wt.-% determined according to method (1) as disclosed herein directly after filling the pharmaceutical composition into a container, "ct(oxytocin)" is the concentration of oxytocin in said pharmaceutical composition in wt.-% determined according to method (1) after storage at 40 °C / 75 % rH for a time "t", and wherein "t" is the time of storage of said pharmaceutical composition at 40 °C / 75 % rH.

It is understood that the time "t" begins directly after filling the pharmaceutical composition into a container, withdrawing a sample for determining c₀(oxytocin), and placing the container comprising said pharmaceutical composition into an environment at a temperature of 40 °C and 75 % relative humidity.

As used herein, the "average increase of impurity C per month" (that may be abbreviated as "AlC") is determined according to the formula (2): $AIC = \frac{{c}_{t} \left(impurity C\right)}{t}$ wherein "ct(impurity C)" is the concentration of impurity C in said pharmaceutical composition in wt.-% determined according to method (1) after storage at 40 °C / 75 % rH for a time "t", and wherein "t" is the time of storage of said pharmaceutical composition at 40 °C / 75 % rH. It is understood that the concentration of impurity C in the pharmaceutical composition in wt.-% determined according to method (1) directly after preparation of said pharmaceutical composition ("c₀(impurity C)") is 0.

It is understood that the time "t" begins directly after filling the pharmaceutical composition into a container, withdrawing a sample for determining c₀(impurity C), and placing the container comprising said pharmaceutical composition into an environment at a temperature of 40 °C and 75 % relative humidity.

As used herein, the "average increase of impurity I per month" (that may be abbreviated as "All") is determined according to the formula (3) $AII = \frac{{c}_{t} \left(impurity I\right)}{t}$ wherein "ct(impurity I)" is the concentration of impurity I in said pharmaceutical composition in wt.-% determined according to method (1) after storage at 40 °C / 75 % rH for a time "t", and wherein "t" is the time of storage of said pharmaceutical composition at 40 °C / 75 % rH. It is understood that the concentration of impurity I in the pharmaceutical composition in wt.-% determined according to method (1) directly after preparation of said pharmaceutical composition ("c₀(impurity I)") is 0.

It is understood that the time "t" begins directly after filling the pharmaceutical composition into a container, withdrawing a sample for determining c₀(impurity I), and placing the container comprising said pharmaceutical composition into an environment at a temperature of 40 °C and 75 % relative humidity.

### Method (1): Determining concentration of oxytocin, impurity I and impurity C in pharmaceutical composition

As used herein, the concentration of oxytocin, impurity C, and impurity I in a pharmaceutical composition is determined according to method (1) as disclosed herein.

For the determination of the amount of oxytocin, the amount of impurity I, and the amount of impurity C, a reversed-phase liquid chromatography is used.

The analysis is performed on a UPLC system using a reverse-phase column (3 x 100 mm, 2.5 µm, P/N 00D-4387-Y0; Phenomex). Elution conditions involve a gradient of binary mobile phases. Mobile phases consist of Solvent A (0.1 % (v/v) formic acid in water for injection) and solvent B (0.1 % (v/v) formic acid in acetonitrile/methanol 1/1 (v/v)) with the following gradient elution programme: 0 to 10 minutes 95/5 to 65/35 A/B, 10 to 13 minutes 65/35 to 40/60 A/B, 13 to 13.2 minutes 40/60 to 95/5 A/B, then reequilibration hold until 16 minutes.

Oxytocin and its degradation products are detected in Single Ion Recording mode with using a mass spectrometer equipped with an electrospray source used in positive mode

Quantification of oxytocin, impurity C and impurity I in wt.-% is performed by external calibration using a pharmacopeial standards (PhEur Oxytocin standard and USP impurity standards, respectively) obtained from commercial supplier.

### Examples

In the following, particular examples illustrating various embodiments and aspects of the invention are presented. However, the present invention shall not to be limited in scope by the specific embodiments described herein. The following preparations and examples are given to enable those skilled in the art to more clearly understand and to practice the present invention. The present invention, however, is not limited in scope by the exemplified embodiments, which are intended as illustrations of single aspects of the invention only, and methods which are functionally equivalent are within the scope of the invention. Indeed, various modifications of the invention in addition to those described herein will become readily apparent to those skilled in the art from the foregoing description, accompanying figures and the examples below. All such modifications fall within the scope of the appended claims.

### Example 1 - Preparation of compositions comprising oxytocin

Compositions comprising oxytocin were prepared as follows (the final composition is indicated in Table 1):
In a production vessel, 90 % (v/v) of the final amount of water for injection was provided at room temperature. Then, sodium chloride (tonicity agent) as indicated in Table 1 and a stabilizer as indicated in Table 1 were dissolved in said water for injection upon stirring for 5 min. After complete dissolution of the tonicity agent and the stabilizer, the pH was adjusted to the final pH as indicated in Table 1 and oxytocin was added. Then, water for injection was added to final volume and the resulting solution was stirred for 10 min. The pH was measured and adjusted to the final pH as indicated in Table 1 again, if needed. The pH and concentration of components (oxytocin and stabilizer) in the composition (i.e. after adding water for injection to final volume) are given in Table 1.

The resulting solution was filtered through a 0.2 µm polyethersulfone membrane filter and filled into plastic bags. The plastic bags were overwrapped in a plastic pouch. All components were purchased from commercial suppliers and are USP grade, unless specified otherwise.

**Table 1 Composition of compositions prepared in example 1**

| **No.** | **Oxytocin final concentration [IU/L]** | **Stabilizer (concentration)** | **final pH** |
|---|---|---|---|
| C1 | 50 | acetate (5 mM) | 3.4 |
| C2 | 50 | acetate (5 mM) | 5.0 |
| C3 | 12 | acetate (5 mM) | 5.0 |
| 1 | 12 | D-lactate (5 mM) | 4.0 |
| 2 | 12 | lysine (5 mM) | 4.0 |
| 3 | 12 | citrate (5 mM) | 4.0 |
| 4 | 50 | citrate (2 mM) | 4.0 |
| | | lysine (5 mM) | |
| 5 | 50 | D-lactate (5 mM) | 4.0 |
| 6 | 50 | lysine (5 mM) | 4.0 |
| 7 | 50 | citrate (5 mM) | 4.0 |

### Example 2 - Stability test at 40 °C

For analyzing the storage stability of the compositions according to example 1, the plastic bags overwrapped with a plastic pouches were stored at 40 °C / 75 % relative humidity. The average loss of oxytocin per month was determined as disclosed herein. The concentration of oxytocin after filling was determined according to method (1) as disclosed herein (c₀(oxytocin)). Samples were withdrawn at different time points, such as about once per month, for up to 8.5 months and the concentration of oxytocin (cₜ(oxytocin)), and optionally the concentration of impurity C and/or impurity I were determined according to method (1). From these data, an average loss of oxytocin per month based on the concentration of oxytocin after filling (C₀) was determined according to formula (1). Furthermore, the average increase of impurity C and impurity I per month was determined according to formula (2) or formula (3), respectively. Results are shown in Table 2.

**Table 2 Results of stability tests of example 2**

| **No.** | **Average loss of oxytocin [wt.-% per month]** | **Average increase of impurity I [wt.-% per month]** | **Average increase of impurity C [wt.-% per month]** |
|---|---|---|---|
| C1 | 5.7 | n.d. | n.d. |
| C2 | 8.6 | n.d. | n.d. |
| C3 | 8.5 | n.d. | n.d. |
| 1 | 2.0 | 0.87 | 0.57 |
| 2 | 1.7 | 0.61 | 0.39 |
| 3 | 2.5 | 0.54 | 0.36 |
| 4 | 1.9 | n.d. | n.d. |
| 5 | 2.4 | 0.88 | 0.57 |
| 6 | 2.3 | 0.50 | 0.33 |
| 7 | 2.6 | 0.59 | 0.40 |

| | | | |
|---|---|---|---|
| n.d.: not determined | | | |

As is concluded from the results of Table 2, the compositions according to the present invention show a superior storage stability as evidenced by the low loss of oxytocin. Also, the formation of impurities C and I is very slow.

Based on the degradation rate of the oxytocin solutions comprising 12 IU/L or 50 IU/L, respectively with the stabilizer as disclosed herein, it can be inferred that after 3 months at 40 °C, the oxytocin solution retains more than 90 % of its original concentration. Using the Arrhenius equation this relates to 12 months of storage at 25 °C.

Therefore, a pharmaceutical composition comprising oxytocin according to the present invention has a shelf life of at least 12 months, thereby maintaining its efficacy and safety for therapeutic use.

### Example 3 - Stability test at different pH

For analyzing the pH dependence of the storage stability of the ready-to-use pharmaceutical compositions according to the present disclosure, a solution consisting of 50 IU/L of oxytocin, 5 mM lysine or without stabilizer, and NaCl as tonicity agent were prepared according to example 1, and the pH was adjusted as shown in Table 3. The resulting solution was filtered through a 0.2 µm membrane polyethersulfone filter and filled into plastic bags. The plastic bags were overwrapped in a plastic pouch. All components were purchased from commercial suppliers and are USP grade, unless specified otherwise.

The plastic bags overwrapped with a plastic pouch were stored at 40 °C / 75 % relative humidity as described in example 2. The concentration of oxytocin was determined by method (1) as disclosed herein and the average loss of oxytocin was determined according to formula (1). Results are shown in Table 3.

**Table 3 Results of stability tests of example 3**

| **No.** | **stabilizer** | **pH** | **Average loss of oxytocin [wt.-% per month]** |
|---|---|---|---|
| C4 | - | 3.0 | 8.3 |
| C5 | - | 4.0 | 4.7 |
| C6 | - | 5.0 | 15.0 |
| 5 | Lysine (5 mM) | 4.0 | 2.3 |

As is concluded from the results of Table 3, the compositions according to the present invention show a superior storage stability as evidenced by the low loss of oxytocin. In particular, a pH of about 4.0 is advantageous for storage stability.

Again, it can be inferred that after 3 months at 40 °C, the oxytocin solution will retain more than 90 % of its original concentration. Using the Arrhenius equation this relates to 12 months of storage at 25 °C.

Therefore, a pharmaceutical composition comprising oxytocin according to the present invention has a shelf life of at least 12 months, thereby maintaining its efficacy and safety for therapeutic use

## Claims

1. A pharmaceutical composition comprising
a) oxytocin;
b) a stabilizer, wherein the stabilizer is selected from the group consisting of
i) lysine or a pharmaceutically acceptable salt thereof,
ii) lactic acid or a pharmaceutically acceptable salt thereof,
iii) citric acid or a pharmaceutically acceptable salt thereof, and
iv) a mixture thereof; and
c) an aqueous medium.

2. The pharmaceutical composition according to claim 1, wherein the pharmaceutical composition has a pH in the range of from about 3.4 to about 6.0, preferably in the range of from about 3.6 to about 5.0, more preferably in the range of from about 3.8 to about 4.5, even more preferably in the range of from about 3.9 to about 4.2, most preferably wherein the pharmaceutical composition has a pH of about 4.0.

3. The pharmaceutical composition according to claim 1 or 2, wherein the stabilizer is lysine or a pharmaceutically acceptable salt thereof.

4. The pharmaceutical composition according to any one of claims 1 to 3, wherein the pharmaceutical composition comprises the stabilizer in a concentration of about 0.5 mM or more, preferably about 1.0 mM or more, more preferably about 2.0 mM or more, even more preferably about 3.0 mM or more, yet more preferably about 4.0 mM or more, most preferably about 4.5 mM or more.

5. The pharmaceutical composition according to any one of claims 1 to 4, wherein the pharmaceutical composition comprises the stabilizer in a concentration of about 50 mM or less, preferably about 30 mM or less, more preferably about 20 mM or less, even more preferably about 10 mM or less, yet more preferably about 8.0 mM or less, most preferably about 6.0 mM or less.

6. The pharmaceutical composition according to any one of claims 1 to 5, wherein the pharmaceutical composition comprises the stabilizer or a pharmaceutically acceptable salt thereof in a concentration in the range of from about 0.5 mM to about 50 mM, preferably in the range of from about 1.0 mM to about 30 mM, more preferably in the range of from about 2.0 mM to about 20 mM, even more preferably in the range of from about 3.0 mM to about 10 mM, yet more preferably in the range of from about 4.0 mM to about 8.0 mM, further more preferably in the range of from about 4.5 mM to about 6.0 mM, most preferably wherein the pharmaceutical composition comprises the stabilizer in a concentration of about 5.0 mM.

7. The pharmaceutical composition according to any one of claims 1 to 6, wherein the pharmaceutical composition comprises oxytocin in a concentration of about 200 IU/L or less, preferably of about 100 IU/L or less, more preferably in a concentration of about 60 IU/L or less, most preferably in a concentration of about 50 IU/L or less.

8. The pharmaceutical composition according to any one of claims 1 to 7, wherein the pharmaceutical composition comprises oxytocin in a concentration of about 5 IU/L or more, preferably in a concentration of about 10 IU/L or more, most preferably in a concentration of about 12 IU/L or more.

9. The pharmaceutical composition according to any one of claims 1 to 8, wherein the pharmaceutical composition comprises oxytocin in a concentration in the range of from about 5 IU/L to about 200 IU/L, preferably in the range of from about 5 IU/L to about 100 IU/L, preferably in a concentration in the range of from about 10 IU/L to about 60 IU/L, more preferably in in a concentration in the range of from about 12 IU/L to about 60 IU/L, most preferably in a concentration in the range of from about 12 IU/L to about 50 IU/L.

10. The pharmaceutical composition according to any one of claims 1 to 9, wherein the pharmaceutical composition further comprises a tonicity agent.

11. The pharmaceutical composition according to claim 10, wherein the tonicity agent comprises a pharmaceutically acceptable alkali metal salt, or a pharmaceutically acceptable earth alkali metal salt, or a monosaccharide, or a mixture thereof, preferably wherein the tonicity agent is selected from the group consisting of sodium chloride, potassium chloride, sodium lactate, calcium chloride, dextrose, and mixtures thereof, more preferably wherein the tonicity agent is sodium chloride, or Ringer's lactate, or dextrose, most preferably wherein the tonicity agent is sodium chloride.

12. The pharmaceutical composition according to any one of claims 1 to 11, wherein the pharmaceutical composition has an osmolarity of about 600 mOsmol/L or less, preferably wherein the pharmaceutical composition has an osmolarity in the range of from about 32 mOsmol/L to about 600 mOsmol/L, more preferably in the range of from about 100 mOsmol/L to about 600 mOsmol/L, more preferably of from about 120 mOsmol/L to about 500 mOsmol/L, more preferably of from about 150 mOsmol/L to about 450 mOsmol/L, more preferably of from about 200 mOsmol/L to about 400 mOsmol/L, more preferably of from about 230 mOsmol/L to about 380 mOsmol/L, more preferably of from about 250 mOsmol/L to about 350 mOsmol/L, most preferably of from about 270 mOsmol/L to about 310 mOsmol/L.

13. The pharmaceutical composition according to any one of claims 1 to 12, wherein the pharmaceutical composition does not comprise chlorobutanol, and/or gluconic acid or a pharmaceutically acceptable salt thereof, and/or glutamic acid or a pharmaceutically acceptable salt thereof, and/or lactobionic acid or a pharmaceutically acceptable salt thereof, and or sucrose, preferably wherein the pharmaceutical composition does not comprise a C₄-C₁₂ carboxylic acid or pharmaceutically acceptable salt thereof, and/ or a disaccharide, and/or a sugar alcohol, and/or chlorobutanol.

14. A method of manufacturing a pharmaceutically acceptable composition according to any one of claims 1 to 13, the method comprising the steps of
a) providing an aqueous medium;
b) dissolving the stabilizer in said aqueous medium to provide a stabilizer solution;
c) adjusting the pH of the stabilizer solution to a pH in the range of from about 3.4 to about 6.0 to provide an acidified stabilizer solution;
d) dissolving oxytocin in said acidified stabilizer solution to provide an oxytocin solution; and
e) sterilizing said oxytocin solution to provide a pharmaceutical composition according to any one of claims 1 to 13.

15. A unit dosage form comprising the pharmaceutical composition according to any one of claims 1 to 13 or a pharmaceutical composition prepared by the method according to claim 14.
